# EUROPEAN PATENT APPLICATION

(11) **EP 4 247 122 A1**
(43) Date of publication of application: **20.09.2023**
(21) Application number: 21926831.5
(22) Date of filing: 28.12.2021
(51) Int. Cl.: H05B 47/155, A61L 2/10, H05B 47/11, H05B 47/16

(54) **METHOD FOR INACTIVATING BACTERIA OR VIRUS**

(30) Priority: 17.02.2021 JP 2021023089
(71) Applicant: Ushio Denki Kabushiki Kaisha, Tokyo 100-8150 (JP)
(72) Inventor: NAITO, Keisuke, Tokyo 100-8150 (JP)
(74) Representative: Tomerius, Isabel
(86) International application number: PCT/JP2021/048817
(87) International publication number: WO 2022/176408

(57) **Abstract**

Provided is a method of inactivating bacteria and/or viruses in a target space using ultraviolet light having wavelengths less than 240 nm, while suppressing the progress of deterioration of a plant. The present invention is a method of inactivating bacteria and/or viruses in a target space where a plant is placed, the method includes a process (a) of irradiating the target space with ultraviolet light having light intensity in a wavelength band belonging to within a range from 190 nm to 235 nm from an ultraviolet light source during a time period when the plant is irradiated with sunlight or visible light from an illumination light source.

## Description

### TECHNICAL FIELD

The present invention relates to a method of inactivating bacteria and/or viruses.

### BACKGROUND ART

Microorganisms (bacteria, fungi, etc.) and/or viruses present in a space or on the surface of an object can cause infectious diseases in humans and non-human animals, and there is a concern that the spread of infectious diseases may threaten people's lives. In particular, infectious diseases are more likely to spread in facilities where people frequently gather, such as medical facilities, schools, and government offices, or vehicles, such as automobiles, trains, buses, airplanes, and ships, thereby necessitating an effective method of inactivating bacteria and/or viruses.

Conventionally, ultraviolet light irradiation has been known as a method of inactivating bacteria and/or viruses (hereinafter collectively referred to as "pathogens"). DNA exhibits the highest absorption characteristic near a wavelength of 260 nm. A low-pressure mercury lamp has a high emission spectrum near a wavelength of 254 nm. Hence, a technology of sterilizing using a low-pressure mercury lamp has been widely adopted.

However, ultraviolet light with this wavelength band is known to adversely affect human bodies when irradiated to humans. The skin is divided into three parts starting from the part near the front surface: epidermis, dermis, and deeper subcutaneous tissue. The epidermis is further divided into four layers starting from the part near the surface: stratum corneum, granular layer, spinous layer, and basal layer. When radiated to human bodies, ultraviolet light having a wavelength of 254 nm penetrates the stratum corneum and reaches the granular layer and the spinous layer, and possibly the basal layer, where it is absorbed by the DNA of cells present in these layers. This results in the risk of skin cancer. Hence, it is difficult to actively use ultraviolet light having the wavelength band in places where people can be present.

Patent Document 1 below discloses that ultraviolet light having a wavelength of 240 nm or longer (UVC light) is harmful to human bodies, and that ultraviolet light having wavelengths of less than 240 nm has a limited degree of adverse effects on human bodies compared to ultraviolet light having a wavelength of 240 nm or longer. In addition, the results of irradiation experiments at wavelengths of 207 nm and 222 nm are specifically described.

### CITATION LIST

Patent Document 1: Japanese Patent No. 6025756
Patent Document 2: JP-A-2009-261289

### SUMMARY OF THE INVENTION

### Technical Problem

The present inventor has studied intensively the problems that may arise when inactivating bacteria and/or viruses in a space, such as a room, using ultraviolet light having wavelengths less than 240 nm, which is considered to have little effect on human bodies. As a result, the inventor newly discovered the problem that a plant in a space may wither due to the irradiation of ultraviolet light having a similar wavelength band in the space when the plant is present.

It is assumed that plants (e.g., foliage plants) may be placed in a target space where the inactivation of bacteria and/or viruses is performed. For example, foliage plants are often placed in conference rooms and offices at workplaces and waiting rooms at medical facilities. When such a space is irradiated with ultraviolet light having wavelengths less than 240 nm to inactivate bacteria and/or viruses, the plants in the space are also irradiated with the ultraviolet light. At this time, although the adverse effects on human bodies associated with the inactivation treatment are suppressed, the ultraviolet light may cause the plants to wither. If such a situation exists, users lose their motivation of introducing inactivation treatment using the ultraviolet light, thereby failing to achieve the effects of controlling the spread of infectious diseases, which is socially undesirable.

On the other hand, as a method to prevent plants from withering, it is conceivable to decrease the irradiation dose within a predetermined time; however, such a method may be insufficiently effective in inactivating the bacteria and/or viruses in the space in the first place.

In view of the above problems, it is an object of the present invention to provide a method of inactivating bacteria and/or viruses in a target space using ultraviolet light having wavelengths less than 240 nm, when a plant is placed in the space while suppressing the progress of deterioration of the plant.

### Problem to be solved

The present invention is a method of inactivating bacteria and/or viruses in a target space where a plant is placed, the method includes a process (a) of irradiating the target space with ultraviolet light having light intensity in a wavelength band belonging to within a range from 190 nm to 235 nm from an ultraviolet light source, and the process (a) is performed during a time period when the plant is irradiated with sunlight or visible light from an illumination light source.

In the present specification, the term "inactivation" refers to a concept that encompasses eliminating bacteria and/or viruses or causing them to lose their infectivity or toxicity, and the term "pathogens" refers to microorganisms such as bacteria and fungi (mold). Hereinafter, "bacteria and/or viruses" may also be collectively referred to as "pathogens".

The use of ultraviolet light having light intensity in the wavelength band belonging to a range from 190 nm to 235 nm suppresses adverse effects on human bodies in terms of inactivating pathogens present in the target space. In addition, irradiating a plant present in the target space with the ultraviolet light with the wavelength band during a time period when the plant is irradiated with sunlight or visible light from an illumination light source makes it possible to suppress the deterioration of the plant. The reason for this will be described below in the section "DESCRIPTION OF EMBODIMENTS".

Meanwhile, a technique for preventing plant disease by ultraviolet light is known (see Patent Document 2 above). This technique utilizes a phenomenon of preventing plant disease by using ultraviolet light with wavelengths from 280 nm to 320 nm, which is generally referred to as the UVB band. More specifically, radiating light with the UVB band to a plant acts on the genes of the plant, causing them to develop hereditary characteristics that are highly protective against disease, thus preventing plant disease. Patent Document 2 describes that ultraviolet light with wavelengths from 280 to 340 nm is used; then it is conceivable that the above principle is utilized when the wavelengths substantially include the UVB band.

Patent Document 2 describes that the irradiation of a plant with the UVB light for preventing diseases in the plant causes the plant to get leaf burn, and that the irradiation of plant with the UVB light in the case of a high illuminance of sunlight can prevent leaf burn.

When plants are irradiated with ultraviolet light to prevent plant disease, ultraviolet light with wavelengths less than 240 nm is not used. There are two reasons for this. The first reason is that, since the irradiation of the UVB light causes leaf burn on plants, the irradiation of plants with ultraviolet light having wavelengths less than 240 nm, which has a shorter wavelength and higher light energy than the UVB light, is predicted to cause leaf burn before achieving the objective of preventing disease. The second reason is that it is unknown whether genes that are highly protective against disease are expressed when plants are irradiated with ultraviolet light having wavelengths less than 240 nm to begin with.

The content of the present invention is not the radiation of ultraviolet light having the UVB band toward a plant to prevent plant disease, but is the radiation of ultraviolet light to inactivate pathogens present in a space. In other words, since ultraviolet light is intended to radiate over a wide area in the space, the illuminance of the ultraviolet light radiated to a plant is lower than that of the UVB band radiated to the a plant for disease control, even if the plant are present in the space. Hence, it was not expected that the plant withers when it is irradiated with ultraviolet light having wavelengths less than 240 nm for the purpose of inactivating pathogens in the space, even if the plant is placed in the space. This is a fact newly discovered by the present inventor as a result of diligent research.

Furthermore, when the inactivation of pathogens present in a space using ultraviolet light having wavelengths less than 240 nm is considered, it is assumed that target spaces can be places where people tend to gather such as automobiles, trains, and movie theaters, for example. And plants are not generally present in these places. In other words, the problem itself such that the phenomenon in which plants wither occurs, when the act of inactivating pathogens present in the space using ultraviolet light having wavelengths less than 240 nm is performed, is an issue newly discovered by the present inventor as a result of diligent research.

According to the present inventor's diligent research, it was newly found that irradiating a target space with ultraviolet light having light intensity in a wavelength range from 190 nm to 235 nm during a time period when the target space is irradiated with visible light can prevent a plant from withering, even when the plant is present in the target space. In other words, according to the present invention, even when the plant is present in the target space, it is possible to inactivate pathogens present in the target space while preventing the plant from withering. Hence, the present invention allows even business persons who have a plant in their offices to be likely to perform the inactivation of bacteria and/or viruses using ultraviolet light having light intensity in a wavelength range from 190 nm to 235 nm. This is expected to have an excellent effect on society in terms of preventing the spread of infectious diseases.

In addition, the ultraviolet light utilized in the method of the present invention does not cause erythema and keratitis on the skin and eyes of humans or animals, and can provide the capabilities of disinfection and inactivation of viruses that are inherent to ultraviolet light. In particular, unlike conventional ultraviolet light sources, the ultraviolet light source can be used in a manned environment, thus installing the ultraviolet light source in indoor and outdoor manned environments enables the entire environment to be irradiated, providing virus control and disinfection in the air and on the surfaces of components placed in the environment. This addresses Goal 3 of the UN-led Sustainable Development Goals (SDGs): "Ensuring healthy lives and promote welfare for all people of all ages" and contributes significantly to Target 3.3 "By 2030, end the epidemics of AIDS, tuberculosis, malaria and neglected tropical diseases and combat hepatitis, water-borne diseases, and other communicable diseases".

By changing an irradiation amount of sunlight or visible light from the illumination light source to the target space with time, there exists a bright environment time period during which the illuminance in the target space is 100 Ix or more, and a dark environment time period during which the illuminance in the target space is less than 100 Ix. The method of inactivating bacteria and/or viruses may include a process (b) of turning on the illumination light source at any time during the dark environment time period, after the bright environment time period is transitioned to the dark environment time period, and the process (a) may be performed after the process (b).

When the target space is, for example, a conference room, an office, a waiting room, a hallway, etc., the target space may have high illuminance during daytime hours because sunlight and visible light from illumination light sources installed in the building enter the target space. In contrast, during nighttime hours, the target space may have low illuminance because no sunlight enters the target space and illumination light sources may be unlit because it is outside office hours. Accordingly, even in the same target space, there may be a case in which a time period with relatively high illuminance (bright environment time period) and a time period with relatively low illuminance (dark environment time period) exist.

The above description gives the case in which the bright environment time period is daytime and the dark environment time period is nighttime as an example; however, this is not limited to this case. For example, in the case in which the target space is completely shielded from outdoor sunlight, the time period when the installed illumination light source is lit is the "bright environment time period", and the time period when it is unlit is the "dark environment time period".

When the target space is irradiated with ultraviolet light during the dark environment time period to inactivate pathogens present therein, there is a risk of accelerating the deterioration of a plant for the reasons mentioned above. However, in the method described above, since ultraviolet light is radiated after the illumination light source is turned on at any point during the dark environment time period, the illuminance in the target space has been increased at least during the time period when the ultraviolet light is radiated. As a result, the progress of deterioration of plants is suppressed.

A specific example will be given below. When the target space is a meeting room, an office, a waiting room, or a hallway of a building, and the like, the illumination light source is often unlit during the nighttime time period because it is outside office hours. Also, no sunlight enters the target space during this time period. Hence, this time period corresponds to the "dark environment time period". During this nighttime time period, after the illumination light source is temporarily turned on, ultraviolet light is radiated from the ultraviolet light source toward the target space.

Another specific example will be given below. In the case in which the target space is a conference room in a building without sunlight, the illumination light source is unlit during the time period when no conference is held even during the daytime hours. This time period corresponds to the "dark environment time period". Meanwhile, during the time period when a meeting takes place, the illumination light source is usually lit. According to JIS Z 9110: 2010 "General rules of recommended lighting level", the basic illumination requirement is set to 500 Ix for normal visual work, so the illuminance in the target space is 100 Ix or more during the time period when the meeting is held, which corresponds to the "bright environment time period".

After the meeting is finished in the conference room, the illumination light source is turned off, and the room transitions from the "bright environment time period" to the "dark environment time period". The dark environment time period is maintained until the meeting starts again. During this dark environment time period, after the illumination light source is temporarily turned on, ultraviolet light is radiated from the ultraviolet light source to the target space.

The process (b) may be a process of turning on the illumination light source after confirming that a current time belongs to the dark environment time period based on time period information regarding at least one of the bright environment time period and the dark environment time period.

When the dark environment time period is nighttime, the time period information regarding the dark environment time period can be, for example, a time period from sunset to sunrise, or a non-operating time period for a company or other entity having jurisdiction over the target space.

More specifically, the illumination light source may include a memory unit that records the time period information and a clock unit that detects the current time, and the process (b) may be a process of turning on the illumination light source after confirming that the current time detected by the clock unit belongs to the dark environment time period based on the time period information read from the memory unit.

The process (a) may contain a process of irradiating the target space with the ultraviolet light during a predetermined ultraviolet light irradiation time period. The method of inactivating bacteria and/or viruses may include, when at least part of the ultraviolet light irradiation time period belongs to the dark environment time period, a process (c) of substantially turning off the ultraviolet light source after the ultraviolet light irradiation time period elapses and a process (d) of substantially turning off the illumination light source being lit after the process (c).

As an example, the time period when ultraviolet light is radiated for inactivation (ultraviolet light irradiation time period) is determined in advance (e.g., from midnight to 2 a.m.) during the dark environmental time period (here, for convenience, nighttime is assumed). In this example, since the ultraviolet light irradiation time period entirely belongs to the dark environment time period, the illumination light source is turned on before the start time of the ultraviolet light irradiation time period, and then ultraviolet light is radiated from the ultraviolet light source to the target space. When the ultraviolet light irradiation time period ends, the radiation of ultraviolet light from the ultraviolet light source is stopped, and the illumination light source is turned off because it is still during the nighttime time period.

Here, the term "substantially turning off the ultraviolet light source" includes the case in which the output of the ultraviolet light source is reduced to less than 0.1 % of its maximum output, as well as the case in which the ultraviolet light source is turned off completely. In addition, "substantially turning off the illumination light source" includes the case in which the illumination light source is turned on with a low brightness state such that the illuminance in the target space is less than 100 Ix as well as the case in which the illumination light source is turned off completely.

The illumination light source may include a first illumination light source that entirely irradiates the target space with visible light and a second illumination light source that locally irradiates the plant with visible light, the second illumination light source may be configured to be capable of turning on even when the first illumination light source is unlit, and the process (a) may be performed when at least the second illumination light source is lit.

According to this method, for example, when performing the inactivation treatment at nighttime, the progress of deterioration of a plant can be suppressed by irradiating the plant with ultraviolet light while lighting the second illumination light source that locally irradiates the plant with visible light. In other words, since it is not necessary to light the first illumination light source that entirely irradiates the target space with visible light, this method suppresses light from leaking to the outside of the target space and prevents the risk of misleading security guards and others believing that the target space is in a state in which the light has been forgotten to be turned off.

The method of inactivating bacteria and/or viruses may include a process (e) of turning on the second illumination light source when the second illumination light source is unlit at a time when the first illumination light source transitions from a lighting state to an unlit state, and the process (a) may be performed after the process (e).

In addition, the method of inactivating bacteria and/or viruses may include a process (f) of detecting illuminance in the target space, and the process (a) may be a process of turning on the ultraviolet light source when the illuminance detected in the process (f) is equal to or more than a reference value of 100 Ix or more.

As will be described later in "DESCRIPTION OF EMBODIMENTS", even if a plant is present in the target space, the deterioration of the plant due to the irradiation of ultraviolet light will be effectively suppressed under an environment in which a visible light illuminance of 100 Ix or more is achieved. This effect is more pronounced when the illuminance is equal to or more than the reference value of 1000 Ix or more.

When the illuminance detected in the process (f) is less than the reference value, the process (a) may include a process of turning on the ultraviolet light source after turning on the illumination light source or increasing light intensity thereof to achieve the illuminance equal to or more than the reference value.

According to the method of inactivation treatment described above, even in the case in which the inactivation treatment in a target space is desired to be performed during a time period when the illuminance of visible light is lower than the reference value, i.e., during an extremely dark time period, inactivation treatment can be performed while preventing the progress of the deterioration of a plant.

The process (f) may be a process of detecting the illuminance in the target space by means of an illuminance meter, and the process (a) may include a process in which a first controller mounted on the illumination light source controls light output of the illumination light source based on information about the illuminance sent from the illuminance meter.

According to the above method, for example, the target space is a meeting room, an office, a waiting room, etc., and even after the illumination light source installed therein is turned off because it is outside office hours (generally at nighttime), the illumination light source is automatically turned on when the ultraviolet light source is turned on for inactivation treatment, thereby suppressing the progress of deterioration of a plant. In particular, performing inactivation treatment by irradiating the target space with ultraviolet light during a time period when humans do not enter or leave the space makes it possible to perform inactivation treatment even in situations where it is desirable to avoid irradiating human bodies with ultraviolet light as much as possible, such as the presence of people suffering from photosensitivity. However, in the case of the above method, if the ultraviolet light source is always lit, the illumination light source will also be always lit. Therefore, if the illumination light source is not desired to be always lit at nighttime or when humans are not present, it is suitable to control the ultraviolet light source to be lit intermittently. In this case, the illumination light source can be turned on at the time when the ultraviolet light source is turned on.

The method of inactivating bacteria and/or viruses may also include a process (g) of substantially turning off the ultraviolet light source being lit when the illuminance detected in the process (f) is less than the reference value.

During the time period when ultraviolet light is radiated to the target space for inactivation treatment, it is assumed that the illuminance in the target space may become less than the reference value due to reasons such as curtains being closed or illumination in the room (illumination light source) being unlit. In such a case, substantially turning off the ultraviolet light source being lit as in the present method suppresses the deterioration of a plant.

The process (f) may be a process of detecting the illuminance in the target space by means of an illuminance meter, and the process (g) may include a process in which a second controller mounted on the ultraviolet light source controls light output of the ultraviolet light source based on information about the illuminance sent from the illuminance meter.

According to the above-mentioned method, when the illuminance in the target space becomes lower than the reference value, the ultraviolet light source automatically transitions to a substantially unlit state, thereby preventing the progress of deterioration of a plant.

The process (a) may be a process of radiating the ultraviolet light during a time period when the visible light from the illumination light source is radiated, the ultraviolet light source and the illumination light source may be housed in the same enclosure, and the first controller and the second controller may be housed in the enclosure to constitute a common controller.

According to the above configuration, it is possible to perform inactivation treatment in the target space while suppressing the deterioration of a plant by means of a device constituted by a single enclosure. This device is an inactivation device with a lighting function or a lighting device with an inactivation treatment function.

The process (a) may be a process of radiating the ultraviolet light during a time period when the visible light from the illumination light source is radiated, in the case where the illuminance sent from the illuminance meter is equal to or more than the reference value, the process (a) may include a process in which the second controller controls the ultraviolet light source to increase light intensity thereof as well as the first controller controls the illumination light source to increase light intensity thereof.

According to the above method, when the output of the ultraviolet light source is increased to enhance the inactivation effect, the output of the visible light of the illumination light source is also increased, thereby suppressing the progress of deterioration of a plant present in the target space while enhancing the inactivation effect.

### Effects of the Invention

The present invention is capable of inactivating bacteria and/or viruses in the space while suppressing the progress of deterioration of a plant.

### BRIEF DESCRIPTION OF DRAWINGS

FIG. 1 is a schematic view illustrating an example of a scene in which the inactivation method of the present invention is implemented.
FIG. 2 is a block diagram schematically illustrating an example of the configuration of an ultraviolet light source.
FIG. 3 is a schematic view illustrating another example of the scene in which the inactivation method of the present invention is implemented.
FIG. 4 is a block diagram schematically illustrating an example of the configuration of a first illumination light source.
FIG. 5 is a schematic view illustrating yet another example of the scene in which the inactivation method of the present invention is implemented.
FIG. 6 is a block diagram schematically illustrating another example of the configuration of the ultraviolet light source.
FIG. 7 is a schematic view illustrating yet another example of the situation in which the inactivation method of the present invention is implemented.
FIG. 8 is a block diagram schematically illustrating another example of the configuration of the first illumination light source.
FIG. 9 is a schematic view illustrating yet another example of the scene in which the inactivation method of the present invention is implemented.
FIG. 10 is a block diagram schematically illustrating an example of the configuration of an inactivation device including an illumination light source and an ultraviolet light source.
FIG. 11 is a schematic view illustrating yet another example of the scene in which the inactivation method of the present invention is implemented.

### DESCRIPTION OF EMBODIMENTS

Embodiments of a method of inactivating bacteria and/or viruses according to the present invention will be described with reference to the drawings as appropriate. Hereinafter, "method of inactivating bacteria and/or viruses" may be simply abbreviated to "inactivation method".

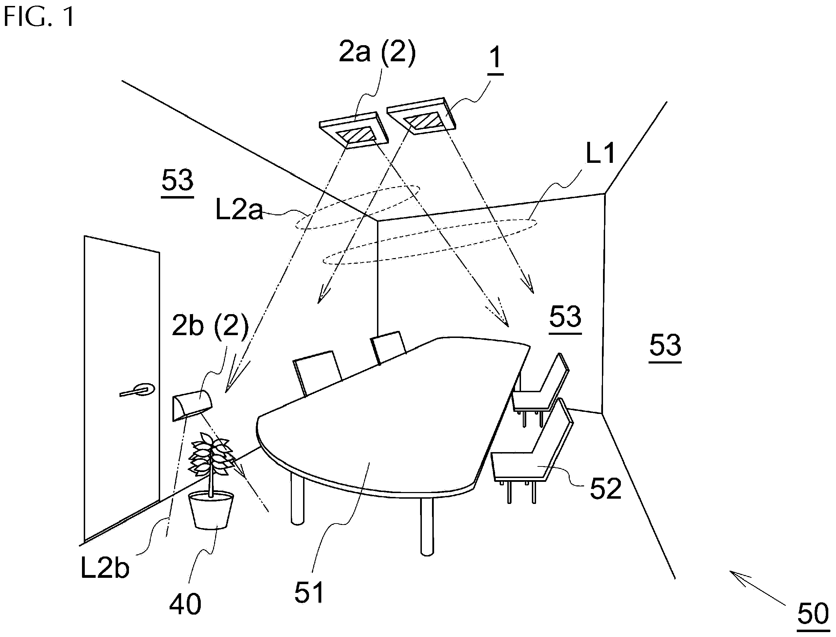

FIG. 1 is a schematic view illustrating an example of a scene in which the inactivation method of the present invention is implemented. The example shown in FIG. 1 illustrates a scene in which the inactivation of pathogens is performed in a room 50, such as a conference room, or, more precisely, to a desk 51, chair 52, and wallpaper 53 installed in the room 50, and the space of the room 50. In other words, in the example shown in FIG. 1, the room 50 corresponds to the "target space".

In the room 50, an ultraviolet light source 1 is installed. The ultraviolet light source 1 is configured to be capable of emitting ultraviolet light L1 that exhibits light intensity in a wavelength band belonging to a range from 190 nm to 235 nm. As an example, the ultraviolet light source 1 is configured to be an excimer lamp filled with a luminescent gas such as KrCl, KrBr, or ArF. The ultraviolet light source 1 needs only to be configured to emit the ultraviolet light L1 with the above wavelengths; thus it can be a solid-state light source such as an LED or LD, or a dielectric barrier discharge lamp in which a luminescent gas is sealed in a light-emitting tube with phosphor coated on its tube wall. The ultraviolet light L1 emitted from the ultraviolet light source 1 may be assumed to exhibit substantially no light intensity in a wavelength band of 240 nm or more. Here, the term "substantially no light intensity" means that the light intensity is less than 5% with respect to the maximum intensity in a wavelength band from 190 nm to 235 nm; and less than 3% is more suitable.

In the room 50, an illumination light source 2a capable of radiating visible light L2a is installed to entirely illuminate the room 50. This illumination light source 2a corresponds to a "first illumination light source" and is referred to as "first illumination light source 2a" as appropriate in the following. The first illumination light source 2a is, for example, a fluorescent lamp or a white LED mounted installed on the ceiling of the room 50.

As shown in FIG. 1, a plant 40 may be installed in the room 50 for an ornamental purpose or the like. In the present embodiment, an illumination light source 2b capable of radiating visible light L2b locally toward the plant 40 is installed. This illumination light source 2b corresponds to a "second illumination light source" and is referred to as "second illumination light source 2b" as appropriate in the following. Both the first illumination light source 2a and the second illumination light source 2b are light sources that radiate visible light and may be collectively referred to as simply "illumination light source 2" when they are not distinguished. In this case, the visible light L2a emitted from the first illumination light source 2a and the visible light L2b emitted from the second illumination light source 2b may be collectively referred to as "visible light L2".

In the present embodiment, the ultraviolet light source 1 radiates the ultraviolet light L1 for inactivation purposes during a time period in which the visible light L2a is radiated to the entire target space (room 50), or during a time period in which no visible light L2a is radiated and the visible light L2b is locally radiated from the second illumination light source 2b to the plant 40.

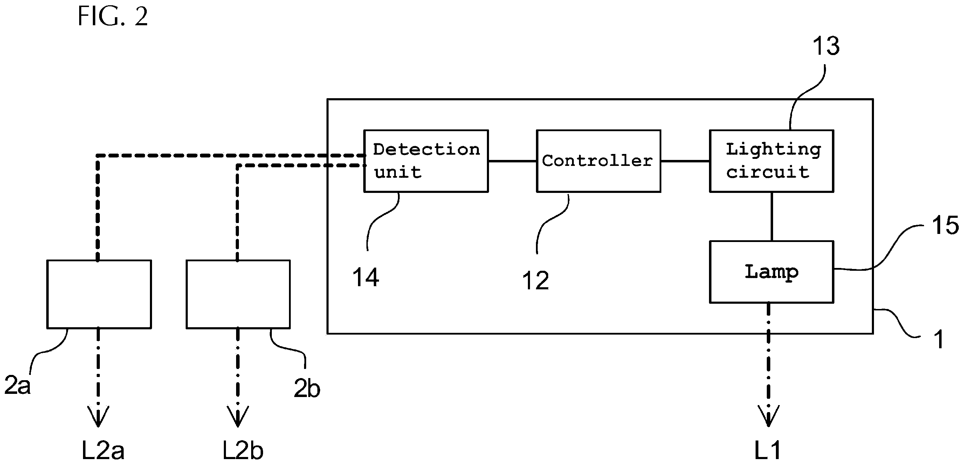

For example, as shown in FIG. 2, the ultraviolet light source 1 includes a lamp 15 that emits the ultraviolet light L1, a lighting circuit 13 that supplies the power necessary for lighting the lamp 15, and a controller 12 that controls the current or voltage supplied to the lamp 15. In the example shown in FIG. 2, the ultraviolet light source 1 includes a detection unit 14 that detects the lighting/unlit state of the illumination light source (2a, 2b).

When the controller 12 performs the control to turn on the lamp 15, it is confirmed in the detection unit 14 that the illumination light sources (2a, 2b) are lit. The detection unit 14 detects whether the illumination light sources (2a, 2b) are lit or not by detecting the energized state of the illumination light sources (2a, 2b), for example. As another example, the detection unit 14 may detect that the illumination light source (2a, 2b) is lit by a signal indicating that the illumination light source (2a, 2b) is lit is transmitted to the detection unit 14.

According to JIS Z 9110: 2010 "General rules of recommended lighting level", the basic illumination requirement is set to 500 Ix for normal vision work. Accordingly, during the time period when the first illumination light source 2a is lit, illuminance equal to or more than 100 Ix is typically achieved in the room 50. This time period corresponds to the "bright environment time period".

In contrast, during the time period when the first illumination light source 2a is unlit, the illuminance in the room 50 is likely to be less than 100 Ix if the room 50 is located in a place where no sunlight enters from the outside, or if it is nighttime in the first place. This time period corresponds to the "dark environment time period".

In the present embodiment, even during the dark environment time period, the visible light (L2a, L2b) is radiated to the plant 40 by lighting the second illumination light source 2b or temporarily lighting the first illumination light source 2a, and ultraviolet light L1 for inactivation is radiated in the room 50 in a state where the irradiation of the visible light with the plant 40 is confirmed. Adoptingthis method is capable of suppressing the progress of deterioration of the plant 40 even when the ultraviolet light L1 is radiated to the plant 40. The reason for this will be described with reference to the experimental results.

A pot-grown white-green Ivy, which is a typical foliage plant, was installed in a conference room that simulates the room 50. The condition of the plant was verified when the ultraviolet light L1 was radiated in the conference room from the ultraviolet light source 1 under the following illumination modes #1 to #3. The respective lighting modes #1 to #3 are shown in Table 1, and the results are shown in Table 2.

**Table 1**

| Illumination mode | Illumination condition on visible light | |
|---|---|---|
| | Indoor fluorescent lamp | Desk light |
| Mode #1 | Lit in daytime only | Unlit |
| Mode #2 | Lit in 24 hours | Unlit |
| Mode #3 | Lit in 24 hours | Lit in nighttime |

**Table 2**

| Elapsed time | Ultraviolet light irradiation with 66 mJ/day | | |
|---|---|---|---|
| | Mode #1 | Mode #2 | Mode #3 |
| 7^{th} day | Leaf burn 10% | Leaf burn 5% | No leaf burn |
| 14^{th} day | Leaf burn 30% | Leaf burn 10% | No leaf burn |
| 21^{st} day | Start to wither | Leaf burn 10% | No leaf burn |
| 28^{th} day | Has withered | Leaf burn 10% | No leaf burn |
| 35^{th} day | - | Leaf burn 15% | No leaf burn |

In both of the modes, a KrCl excimer lamp emitting the ultraviolet light L1 with a peak wavelength of 222 nm was used as the ultraviolet light source 1. As of the filing date of this application, the American Conference of Governmental Industrial Hygienists (ACGIH) and JIS Z 8812 (Methods for Measuring Hazardous Ultraviolet Light Radiation), and other standards, specify the threshold limit value (TLV) for each wavelength with respect to the irradiation amount of ultraviolet light per day (8 hours) to human bodies. According to these standards, an irradiation dose of 22 mJ in 8 hours is defined as the TLV for the ultraviolet light with a wavelength of 222 nm. Accordingly, in the viewpoint of simulating this illuminance, the ultraviolet light L1 was continuously radiated to the room from the ultraviolet light source 1 with an irradiation dose of 66 mJ in 24 hours.

The mode #1 is an illumination mode in which an indoor fluorescent lamp installed in the conference room is lit for 12 hours from 7 am to 7 pm (day time period) and this indoor fluorescent lamp is unlit for 12 hours from 7 pm to 7 am (night time period). In other words, in the case of the mode #1, the ultraviolet light L1 was radiated to the room under a nearly pitch-dark environment during the night time period. As the indoor fluorescent lamp, a fluorescent lamp for general lighting (as per JIS C 7601 :2010) was used. This indoor fluorescent lamp simulated the first illumination light source 2a.

The mode #2 is an illumination mode in which the indoor fluorescent lamp installed in the conference room is lit over 24-hours. In other words, in the case of the mode #2, the ultraviolet light L1 was radiated to the room under a bright environment achieved by the lighting of the indoor fluorescent lamp day and night. The illuminance in the conference room at nighttime in the mode #2 was set to 100 lx (lux).

The mode #3 is an illumination mode in which the indoor fluorescent lamp installed in the conference room is lit for 24 hours, and a desk light is additionally lit to irradiate the foliage plant with visible light from the desk light for 12 hours from 7 p.m. to 7 a.m. (night time period). In other words, in the mode #3, the ultraviolet light L1 radiated to the room in a bright environment achieved by the lighting of the indoor fluorescent lamp day and night, as well as the vicinity of the foliage plant was made brighter than in the mode #2 at nighttime. In the mode #3, the illuminance in the vicinity of the foliage plant in the conference room at nighttime was set to 1000 lx (lux). As the desk light, an illumination device embedded with a white LED element, which exhibits light output in a wavelength range from 420 nm to 800 nm, was utilized. This desk light simulated the second illumination light source 2b.

For each mode #1 to #3, the condition of the foliage plant was checked on the 7th, 14th, 21st, 28th, and 35th day.

As shown in Table 2, in the case of the mode #1, approximately 10% of the foliage plant exhibited leaf burn on the 7th day and began to wither on the 21st day. Furthermore, on the 28th day, the leaves thereof fell to pieces in tatters when touched by hand, and withered completely.

In the case of the mode #2, approximately 5% of the foliage plant exhibited leaf burn on the 7th day, and only approximately 10% thereof exhibited leaf burn on the 28th day. Approximately 15% thereof exhibited leaf burn on the 35th day; however, it failed to reach the state of starting to wither. In the case of the mode #3, no leaf burn was observed on the 35th day, and the foliage plant remained in nearly the same condition as that before the start of the experiment.

When the experiment was conducted with the same conditions except that the brightness of the ultraviolet light source 1 was increased to radiate with an irradiation dose of 100 mJ in 24 hours, the results similar to those in Table 2 were obtained except that the number of elapsed days in which the same phenomenon was observed was generally shortened.

These experimental results led to the conclusion that the irradiation of a plant with the ultraviolet light L1 having a wavelength of 222 nm during the time period when the visible light (L2a, L2b) is not radiated induces an effect that causes the deterioration of the plant to progress, and that the irradiation of a plant with the visible light (L2a, L2b) during the irradiation of the ultraviolet light L1 can suppress the progress of the deterioration of the plant. The present inventor infers that the following is the reasons for this.

First, ultraviolet light with wavelengths from 190 nm to 235 nm including 222 nm produces little effect when radiated to human bodies, unlike ultraviolet light with a wavelength of 254 nm, which is commonly used for disinfection purposes. The reason for this is that, as described above in the "BACKGROUND ART" section, ultraviolet light with a wavelength of 254 nm penetrates the stratum corneum present on the front surface of the epidermis and is absorbed by the DNA of cells in the underlying layer, whereas ultraviolet light with wavelengths from 190 nm to 235 nm hardly penetrates the stratum corneum.

Plants such as foliage plants typically have epidermal cells, and there is a cuticle layer or a wax layer on the outer side of the epidermis, that covers the epidermis to prevent dust, pathogens, or the like from entering the epidermis. Hence, before conducting the experiment, the present inventor thought that the irradiation of a plant with ultraviolet light with wavelengths from 190 nm to 235 nm would have no significant effect on the plant because the ultraviolet light would only be absorbed by the cuticle layer of a plant and would not reach the cell nuclei of a plant, which is the similar reason as that for human bodies. However, as shown in Table 2, the foliage plant actually withered in the case of the mode #1.

Plants (especially leaves) contain a substance called chlorophyll P700. This chlorophyll P700, when given light energy, is excited to P700^{∗} to transfer electrons (e-) to others by oxidation. Here, under the environment where photosynthesis proceeds, electrons (e-) are passed to nicotinamide dinucleotide phosphate (NAPD⁺), resulting in the generation of NADPH. In contrast, under the conditions in which the progress of photosynthesis is suppressed, electrons generated during the oxidation of P700^{∗} are given to oxygen, resulting in the generation of active oxygen. It is assumed that plants wither due to the action of this active oxygen on the plants. This is consistent with the fact that the deterioration of the plant was suppressed in the modes #2 and #3, in which visible light was radiated at nighttime, compared to the mode #1.

As described above, it can be seen that, when the ultraviolet light L1 for inactivation is radiated from the ultraviolet light source 1 to the room 50, detecting the lighting state of the illumination light sources (2a, 2b) in advance enables the inactivation in the room 50 while suppressing the progress of deterioration of the plant 40.

Incidentally, in the case of the time period (bright environment time period) when the first illumination light source 2a is lit, visible light is radiated to the plant 40 without additionally turning on the second illumination light source 2b. In other words, the irradiation of the ultraviolet light L1 during this time period can suppress the progress of deterioration of the plant 40.

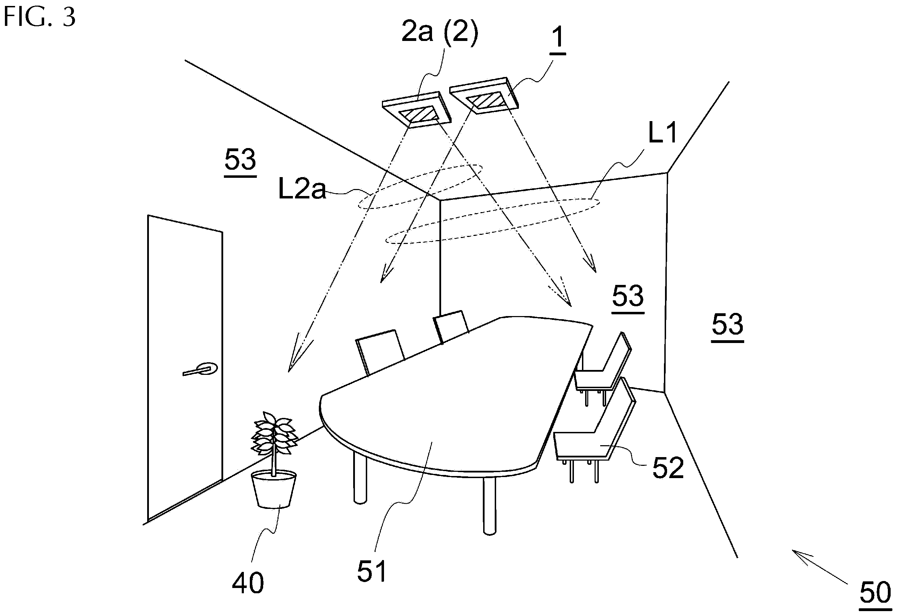

From this viewpoint, as shown in FIG. 3, the second illumination light source 2b may not be provided in the room 50 whereas the first illumination light source 2a is provided therein. In this case, the first illumination light source 2a may be automatically turned off after the completion of irradiation process of the ultraviolet light L1.

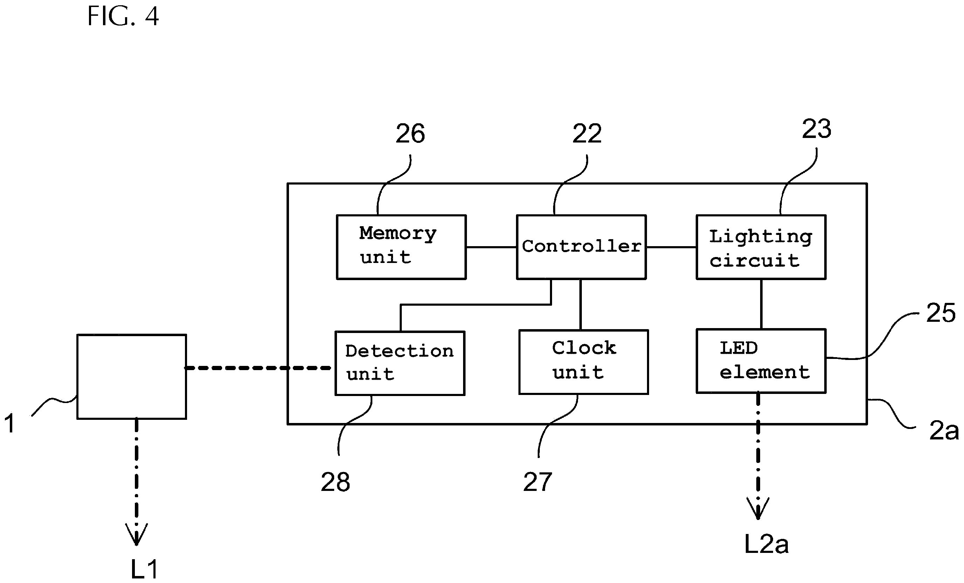

As shown in FIG. 4, the first illumination light source 2a, for example, includes an LED element 25 that emits the white visible light L2a, a lighting circuit 23 that supplies the LED element 25 with the power necessary for lighting, and a controller 22 that adjusts the current or voltage supplied to the LED element 25. In addition, the first illumination light source section 2a also includes a memory unit 26, a clock unit 27, and a detection unit 28.

For example, if the room 50 is an office under the jurisdiction of a company or other entity, there may be a case in which information about the time period when the illuminance in the room 50 is relatively high (bright environment time period) or the time period when the illumination therein is relatively low (dark environment time period) can be recognized in advance. Typically, nighttime and late-night time periods, which are outside office hours, correspond to the dark environment time period, and other time periods correspond to the bright environment time period.

The memory unit 26 includes a memory medium in which time period information relating to at least one of the bright environment time period and the dark environment time period is recorded. The clock unit 27 has a function of detecting the current time and includes an interface that receives the current time from a server (not shown), for example, a clock circuit.

The detection unit 28 detects whether the ultraviolet light source 1 is lit or not. The detection unit 28 detects the lighting state of the ultraviolet light source 1 by detecting the energized state of the ultraviolet light source 1 or by detecting a signal from the ultraviolet light source 1 indicating of being lit.

Suppose that during the time period when the illumination light source 2a is lit, the ultraviolet light L1 is radiated from the ultraviolet light source 1 to the room 50 to perform the inactivation treatment. When this inactivation treatment is completed, the ultraviolet light source 1 is turned off. Detecting that the ultraviolet light source 1 becomes unlit, the detection unit 28 sends a signal indicating the status to the controller 22. Since the ultraviolet light source 1 may be lit periodically or intermittently during the inactivation treatment, the detection unit 28 may send the signal indicating the status to the controller 22 when it detects that the ultraviolet light source 1 has been unlit continuously for a predetermined time or longer (e.g., 20 minutes or longer).

Receiving a notification from the detection unit 28 that the ultraviolet light source 1 is unlit, the controller 22 reads the time period information from the memory unit 26 and identifies whether the current time detected by the clock unit 27 is during the bright environment time period or the dark environment time period. If the current time is during the dark environment time period, the controller 22 controls the lighting circuit 23 to turn off the LED element 25. This eliminates the situation where the first illumination light source 2a continues to be lit during the dark environment time period where no inactivation treatment is performed. This controller 22 corresponds to a "first controller".

In the case where the ultraviolet light L1 is radiated from the ultraviolet light source 1 during a predetermined time period (ultraviolet light irradiation time period), information on the ultraviolet light irradiation time period may be recorded in the memory unit 26. In this case, the controller 22 may continuously allow the LED element 25 to be lit from before a predetermined time (e.g., before 5 minutes) prior to the start time of the ultraviolet light irradiation time period to after a predetermined time (e.g., 5 minutes after) from the end time of the ultraviolet light irradiation time period.

If the end time of the ultraviolet light irradiation time period belongs to the dark environment time period, the controller 22 may automatically turn off the LED element 25 after a predetermined time (5 minutes in the above example) from the end time.

The example shown in FIG. 4 describes the case in which the first illumination light source 2a includes the LED element 25; however, other light sources, such as a fluorescent lamp, can be used as long as the controller 22 can perform the lighting control.

As described above, it can be seen that by irradiating the plant 40 with the visible light L2 (L2a, L2b) from the illumination light source 2 (2a, 2b) when the ultraviolet light L1 for inactivation is radiated from the ultraviolet light source 1 to the room 50, this makes it possible to perform the inactivation in the room 50 while suppressing the progress of deterioration of the plant 40.

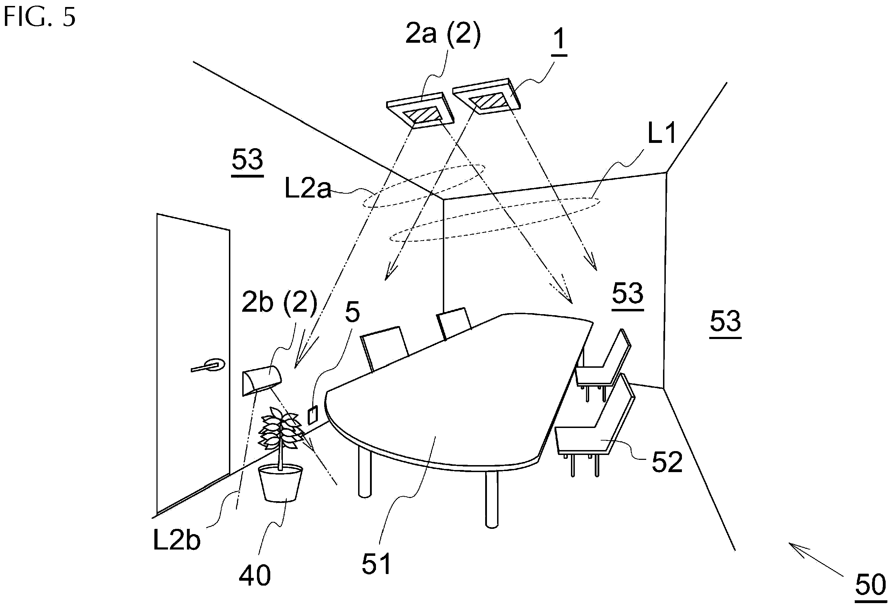

As another aspect, as shown in FIG. 5, the illuminance in the room 50 may be measured by an illuminance meter 5 installed in the room 50, and the dose of the ultraviolet light L1 from the ultraviolet light source 1 can be adjusted based on the results of this measurement.

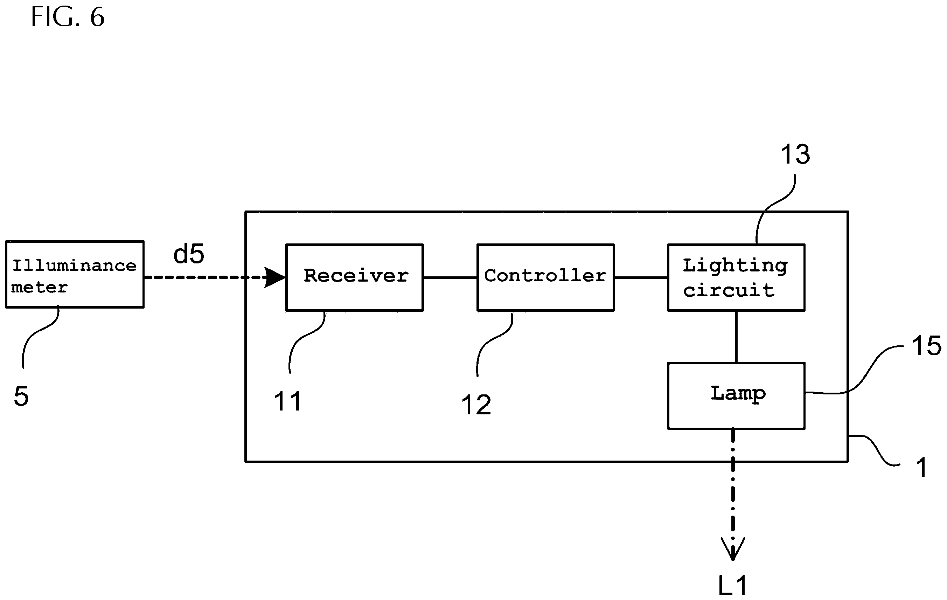

FIG. 6 is a block diagram schematically illustrating another example of the configuration of the ultraviolet light source 1. The ultraviolet light source 1 shown in FIG. 6 includes a receiver 11 that receives illuminance information d5 from the illuminance meter 5, the lamp 15 that emits the ultraviolet light L1, the lighting circuit 13 that supplies the power necessary for the lighting of the lamp 15, and the controller 12 that adjusts the current or voltage supplied to the lamp 15. The controller 12 is a control means for transmitting a control signal to the lighting circuit 13 and includes a processor, such as a CPU, and a memory for storing information. The controller 12 receives the illuminance information d5 that has been received at the receiver 11. In FIG. 6, the controller 12 corresponds to a "second controller".

Based on the illuminance information d5, the controller 12 outputs an instruction signal to the lighting circuit 13 to substantially turn off the lamp 15 when the illuminance measured by the illuminance meter 5 is less than the reference value. In this case, the controller 12 may maintain the information regarding the reference value in the memory. When the illuminance of the visible light L2 radiated to the plant 40 is low, such a configuration allows the irradiation of the ultraviolet light L1 to be substantially stopped, thereby suppressing the deterioration of the plant 40.

With the results of the experiment described above considered, the reference value can be set to 100 Ix as an example. In this case, if the illuminance of the visible light L2 radiated to the plant 40 is less than 100 lx, the irradiation of the ultraviolet light L1 may be stopped. Note that an increase in the value of this reference value will enhance the effect of suppressing the progress of deterioration of the plant 40. However, in the case of this control method, if the reference value is set too high, the ultraviolet light source 1 will hardly be lit in the first place, thereby possibly failing to achieve the inactivation effect.

According to JIS Z 9110: 2010 "General rules of recommended lighting level", the basic illumination requirement is recommended to be 500 lx for normal visual work, 1000 lx for precision visual work, and 2000 lx for ultra-precision visual work. Accordingly, the upper limit of the reference value is preferably set to 2000 lx or less, and typically it is more preferably set to 1000 lx or less.

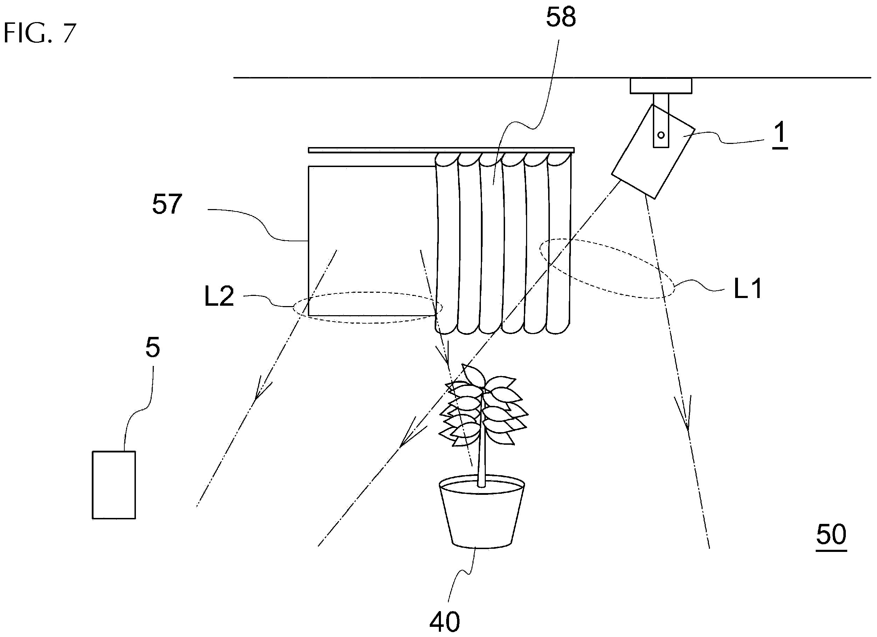

From the above viewpoint, there is no need for the illumination light source 2 in the room 50. For example, as shown in FIG. 7, in the case where sunlight 57 can be radiated to the room 50 by opening a curtain 58, the deterioration of the plant 40 can be suppressed by radiating the ultraviolet light L1 from the ultraviolet light source 1 when the illuminance is above the reference value due to the presence of the sunlight 57. On the other hand, in the case where the illuminance of the room 50 detected by the illuminance meter 5 indicates less than the reference value because the curtain 58 is closed or for other reasons, the ultraviolet light source 1 may be substantially turned off by the controller 12.

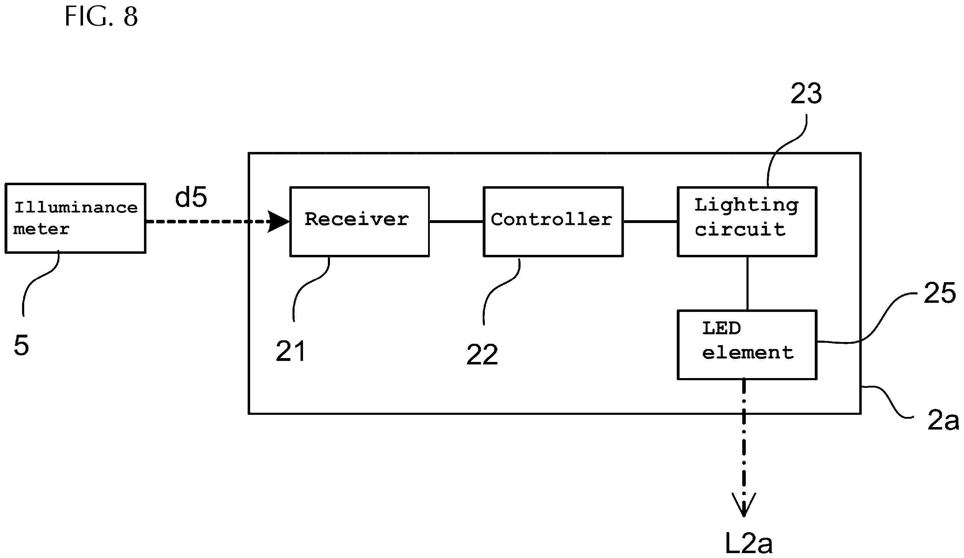

In the aspect shown in FIG. 5, the output of the illumination light sources 2 (2a, 2b) may be controlled based on the illuminance results detected by the illuminance meter 5. FIG. 8 is a diagram schematically illustrating another example of the configuration of the first illumination light source 2a, which includes a receiver 21 that receives the illuminance information d5 from the illuminance meter 5, the LED element 25 that emits the visible light L2a, the lighting circuit 23 that supplies power necessary for lighting the LED element 25, and the controller 22 that adjust the current or voltage supplied to the LED element 25. The controller 22 receives the illuminance information d5 that has been received at the receiver 21.

Based on the illuminance information d5, the controller 22 can output an instruction signal to the lighting circuit 23 to turn on the LED element 25 or increase the brightness of the LED element 25 when the illuminance measured by the illuminance meter 5 is less than the reference value. In this case, the controller 22 may maintain the information regarding the reference value in the memory. When the illuminance of the visible light radiated to the plant 40 is low, such a configuration allows the irradiation of the visible light L2a from the first illumination light source 2a to be increased, thereby suppressing the deterioration of the plant 40 due to the ultraviolet light L1.

Even when the illuminance meter 5 is installed in the room 50, the second illumination light source 2b may not be installed in the same manner as described above with reference to FIG. 3.

In the above description, the case where the output of the first illumination light source 2a is controlled based on the illuminance measured by the illuminance meter 5 is described. However, in the case where the second illumination light source 2b is installed in the room 50, the output of the second illumination light source 2b may be controlled based on the illuminance measured by the illuminance meter 5.

Furthermore, the controller 22 may control both of the ultraviolet light source 1 and the illumination light sources 2 (2a, 2b). For example, the controller 12 of the ultraviolet light source 1 (see FIG. 6) and the controller 22 of the first illumination light source 2a (see FIG. 8) may control the light output of the light sources (1, 2a), respectively, based on the illuminance information d5 from the illuminance meter 5. The same control may be performed for the second illumination light source 2b instead of the first illumination light source 2a or together with the first illumination light source 2a.

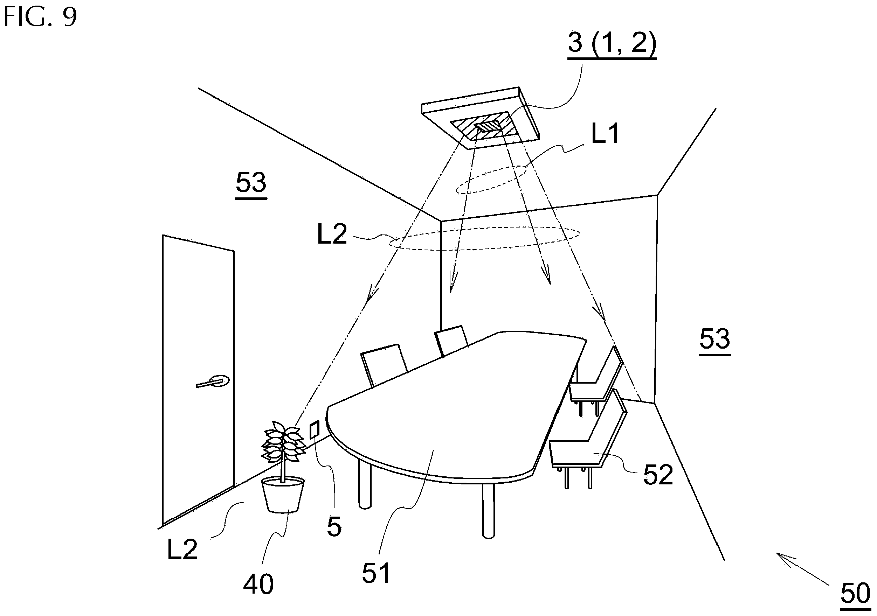

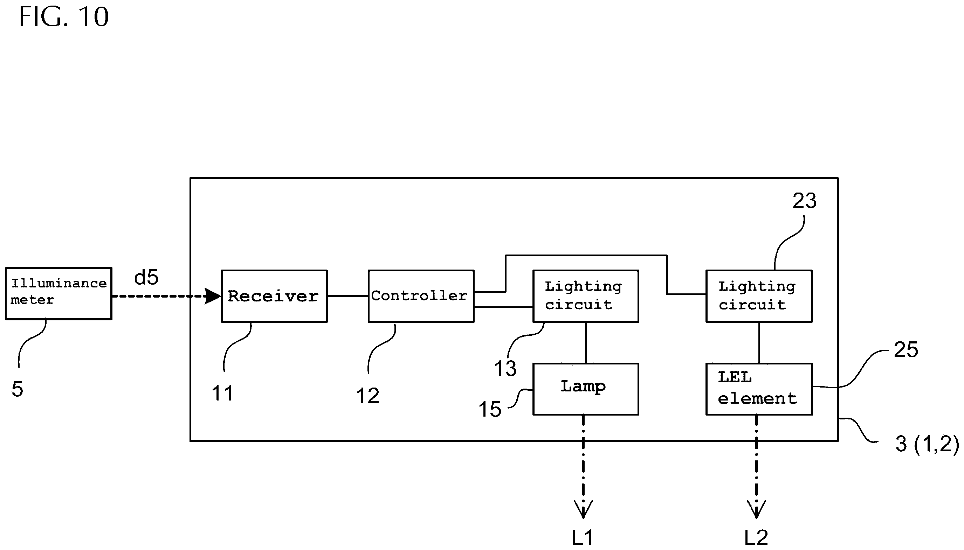

As shown in FIG. 9, an inactivation device 3 that houses the ultraviolet light source 1 and the illumination light source 2 in a common enclosure may perform the inactivation and the visible light illumination in the room 50. In this case, as shown in FIG. 10, the controller 12 may commonly control the light output of the lamp 15 emitting the ultraviolet light L1 and the light output of the LED element 25 emitting the visible light L2.

For enhancing the inactivation effect, the controller 22 may control both of the ultraviolet light source 1 and the illumination light source 2 to increase the respective light outputs. Increasing the intensity of the ultraviolet light L1 can increase the inactivation effect in the room 50, but in this case, the rate of deterioration of the plant 40 may be accelerated. In contrast, increasing the intensity of the visible light L2 in addition to the intensity of the ultraviolet light L1 makes it possible to enhance the inactivation effect while suppressing the progress of deterioration of the plant 40.

### Another embodiment

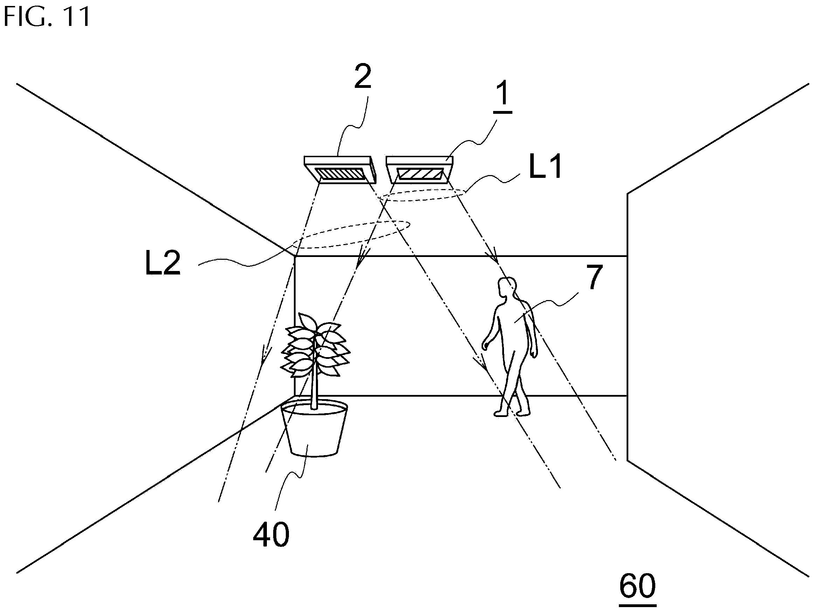

Hereinafter, another embodiment will be described.
<1 > In the above embodiment, white light is assumed as the visible light L2. However, in view of the results of the verification described above, it is considered that visible light that is at least expected to act on a photosynthetic effect on the plant 40 is sufficient. In other words, the visible light L2 may be light that contains at least a component in the blue region (belonging to within a wavelength range from 430 to 500 nm) and a component in the red region (belonging to within a wavelength range from 590 to 700 nm).
<2> In the above embodiment, the room 50 in a building, such as a conference room schematically shown in FIG. 1, is exemplified as a target space for performing the inactivation treatment. However, the target space is not limited to the room 50, and the present invention can be applied to any space where people are expected to enter and leave. For example, as shown in FIG. 11, a target space for inactivation can be a hallway 60 in a building where a person 7 is expected to pass through. FIG. 11 illustrates an example in which the ultraviolet light source 1 and the illumination light source 2 are installed on the ceiling of the hallway 60, and the ultraviolet light L1 and the visible light L2 are radiated from the respective light sources. In this case, the inactivation treatment in the hallway 60 can also be performed while suppressing the progress of deterioration of the plant 40 installed in the hallway 60.
   The target space for inactivation may also be a space where the illumination light source 2 is not installed as described above and where the irradiation of the visible light L2 from the sun is possible. Examples of such a space include vinyl houses.
<3> In the above example, the case where the ultraviolet light source 1 is installed on the ceiling of the room 50 is described; however, the ultraviolet light source 1 may be installed on a wall of the room 50, on the desk 51, or the like. In the present invention, the configuration of installation of the ultraviolet light source 1 is not limited.
<4> Lighting control may be manually performed in each of the ultraviolet light source 1 and the illumination light sources 2 (2a, 2b). In other words, during the dark environment time period, after the illumination light source 2 (2a, 2b) is manually turned on, the ultraviolet light source 1 may be manually turned on. Then, after the ultraviolet light source 1 is manually turned off, the illumination light sources 2 (2a, 2b) may be manually turned off. As described above, the ultraviolet light source 1 is configured to emit the ultraviolet light L1 with wavelengths of less than 240 nm, which suppresses adverse effects on human bodies, hence, even if the person 7 enters the room 50 where the ultraviolet light L1 is radiated in order to turn off the ultraviolet light source 1 or the illumination light source 2, no adverse effect on the human bodies of the person 7 will occur.
   When the lighting control of the ultraviolet light source 1 can be performed by remote control or the like, the ultraviolet light source 1 can be manually turned off without a person 7 entering the room 50 where the ultraviolet light L1 is radiated in the first place.
<5> In the aspect of FIG. 7, the illuminance meter 5 may not be installed in the room 50. In this case, for example, after the person 7 opens the curtain 58 during the day time period, the ultraviolet light source 1 can be turned on manually to suppress the progress of deterioration to the plant 40. After turning off the ultraviolet light source 1, the curtain 58 may be closed again.

### Reference Signs List

- 1: Ultraviolet light source
- 2: Illumination light source
- 2a: First illumination light source
- 2b: Second illumination light source
- 3: Inactivation device
- 5: Illuminance meter
- 7: Person
- 11: Receiver
- 12: Controller
- 13: Lighting circuit
- 14: Detection unit
- 15: Lamp
- 21: Receiver
- 22: Controller
- 23: Lighting circuit
- 25: LED element
- 26: Memory unit
- 27: Clock unit
- 28: Detection unit
- 40: Plant
- 50: Room
- 51: Desk
- 52: Chair
- 53: Wallpaper
- 57: Sunlight
- 58: Curtain
- 60: Hallway
- L1: Ultraviolet light
- L2 (L2a, L2b): Visible light

## Claims

1. A method of inactivating bacteria and/or viruses in a target space where a plant is placed, the method comprising a process (a) of irradiating the target space with ultraviolet light having light intensity in a wavelength band belonging to within a range from 190 nm to 235 nm from an ultraviolet light source,
wherein the process (a) is performed during a time period when the plant is irradiated with sunlight or visible light from an illumination light source.

2. The method of inactivating bacteria and/or viruses according to claim 1, further comprising a process (b) of turning on the illumination light source at any time during a dark environment time period after a bright environment time period is transitioned to the dark environment time period,
wherein the bright environment time period during which the illuminance in the target space is 100 Ix or more, and a dark environment time period during which the illuminance in the target space is less than 100 Ix exist by changing an irradiation amount of sunlight or visible light from the illumination light source to the target space with time, and
the process (a) is performed after the process (b).

3. The method of inactivating bacteria and/or viruses according to claim 2, wherein the process (b) is a process of turning on the illumination light source after confirming that a current time belongs to the dark environment time period based on time period information regarding at least one of the bright environment time period and the dark environment time period.

4. The method of inactivating bacteria and/or viruses according to claim 3, wherein the illumination light source includes a memory unit that records the time period information and a clock unit that detects the current time, and the process (b) is a process of turning on the illumination light source after confirming that the current time detected by the clock unit belongs to the dark environment time period based on the time period information read from the memory unit.

5. The method of inactivating bacteria and/or viruses according to any of claims 2 to 4, wherein the process (a) contains a process of irradiating the target space with the ultraviolet light during a predetermined ultraviolet light irradiation time period,
the method further comprising a process (c) of substantially turning off the ultraviolet light source after the ultraviolet light irradiation time period elapses when at least part of the ultraviolet light irradiation time period belongs to the dark environment time period and a process (d) of substantially turning off the illumination light source being lit after the process (c).

6. The method of inactivating bacteria and/or viruses according to any of claims 1 to 4, wherein the illumination light source includes a first illumination light source that entirely irradiates the target space with visible light and a second illumination light source that locally irradiates the plant with visible light, the second illumination light source is configured to be capable of turning on even when the first illumination light source is unlit, and the process (a) is performed when at least the second illumination light source is lit.

7. The method of inactivating bacteria and/or viruses according to any of claims 2 to 4, wherein the illumination light source includes a first illumination light source that entirely irradiates the target space with visible light and a second illumination light source that locally irradiates the plant with visible light, the second illumination light source is configured to be capable of turning on even when the first illumination light source is unlit, and the process (b) is a process of turning on the second illumination light source at any time during the dark environment time period.

8. The method of inactivating bacteria and/or viruses according to claim 6, the method further comprising a process (e) of turning on the second illumination light source when the second illumination light source is unlit at a time when the first illumination light source transitions from a lighting state to an unlit state, wherein the process (a) is performed after the process (e).

9. The method of inactivating bacteria and/or viruses according to claim 1, the method further comprising a process (f) of detecting illuminance in the target space, wherein the process (a) is a process of turning on the ultraviolet light source when the illuminance detected in the process (f) is equal to or more than a reference value of 100 lx or more.

10. The method of inactivating bacteria and/or viruses according to claim 9, wherein when the illuminance detected in the process (f) is less than the reference value, the process (a) includes a process of turning on the ultraviolet light source after turning on the illumination light source or increasing light intensity thereof to achieve the illuminance equal to or more than the reference value.

11. The method of inactivating bacteria and/or viruses according to claim 10, wherein the process (f) is a process of detecting the illuminance in the target space by means of an illuminance meter, and the process (a) includes a process in which a first controller mounted on the illumination light source controls light output of the illumination light source based on information about the illuminance sent from the illuminance meter.

12. The method of inactivating bacteria and/or viruses according to any one of claims 9 to 11, the method further comprising a process (g) of substantially turning off the ultraviolet light source being lit when the illuminance detected in the process (f) is less than the reference value.

13. The method of inactivating bacteria and/or viruses according to claim 12, wherein the process (f) is a process of detecting the illuminance in the target space by means of an illuminance meter, and the process (g) includes a process in which a second controller mounted on the ultraviolet light source controls light output of the ultraviolet light source based on information about the illuminance sent from the illuminance meter.

14. The method of inactivating bacteria and/or viruses according to claim 13, wherein the process (a) is a process of radiating the ultraviolet light during a time period when the visible light from the illumination light source is radiated, the ultraviolet light source and the illumination light source are housed in the same enclosure, and the first controller and the second controller is housed in the enclosure to constitute a common controller.

15. The method of inactivating bacteria and/or viruses according to claim 13, wherein the process (a) is a process of radiating the ultraviolet light during a time period when the visible light from the illumination light source is radiated, and
in a case where the illuminance sent from the illuminance meter is equal to or more than the reference value, the process (a) includes a process in which the second controller controls the ultraviolet light source to increase light intensity thereof as well as the first controller controls the illumination light source to increase light intensity thereof.
